# EUROPEAN PATENT APPLICATION

(11) **EP 4 512 357 A1**
(43) Date of publication of application: **26.02.2025**
(21) Application number: 24170574.8
(22) Date of filing: 16.04.2024
(51) Int. Cl.: A61B 34/35, A61B 34/37, A61B 34/00, A61M 25/09, A61B 34/30

(54) **A MOTION SYSTEM FOR VASCULAR INTERVENTION NAVIGATION SURGERY**

(30) Priority: 22.08.2023 CN 202311060465; 03.11.2023 CN 202311457616
(71) Applicant: J Robotics Medical Ltd, Shanghai 201613 (CN)
(72) Inventor: Shen, Bifeng, Shanghai, 201103 (CN); Hong, Jiong, Shanghai, 201601 (CN); Shen, Xinzhou, Shanghai, 201613 (CN)
(74) Representative: Neumann, Ditmar

(57) **Abstract**

The present invention discloses a motion system for a vascular intervention navigation surgery system, wherein the motion system is configured to control the motion of the vascular intervention navigation surgery system, and the motion system comprises a support base and the vascular intervention navigation surgery system. The motion system of the present invention provides more degrees of freedom of motion and other functions for the vascular intervention navigation surgery system, guidewires, catheters, stents, etc., making it more convenient to perform vascular intervention surgery. The motion system of the present invention further comprises a force reproduction system for the vascular intervention navigation surgery system. The force reproduction system of the present invention can effectively detect a resistance of the guidewire in blood vessels, and by processing the resistance, determine the further movement status of the guidewire, thereby improving the safety and operational efficiency of vascular intervention surgery.

## Description

### Cross-reference to prior applications

This application claims priority to Chinese Application No. 2023114576167 filed on November 3, 2023 and priority to Chinese Application No. 2023110604651 filed on August 22, 2023, which are incorporated herein by reference in their entirety.

### Technical field

The invention relates to the field of medical devices, and more specifically to a motion system for a vascular intervention navigation surgery system.

### Background

Before performing vascular intervention surgery, the surgical robot needs to be installed on the operating bed. During the vascular intervention surgery process, the vascular intervention surgery is performed by controlling the vascular intervention navigation surgery system and its motion system.

At present, the vascular intervention navigation surgery system is able to control the forward and backward movement of the guidewire, as well as the forward and backward movement of the balloon catheter, stent catheter, and guide catheter, etc., as described in the Chinese invention patent submitted on November 5, 2021, with publication number CN 113598947B and invention name "Vascular Interventional Navigation Surgery system", which is integrated into this application by reference in its entirety. The vascular intervention navigation surgery system is a highly safe robot system that assists doctors in performing vascular intervention surgery, which can avoid serious consequences caused by physiological tremors and misoperation by the doctors during surgery, and can protect the doctors from X-ray radiation. During the surgical process, it may be possible that the doctors may be off-site with the patient and perform the surgery by controlling the vascular intervention navigation surgery system through remote operation. When the guidewire moves in the blood vessel, it will come into contact with the vessel wall and face resistance. Mechanized operation makes it difficult for the doctors to sense the resistance of the guidewire during its movement in the blood vessel. This increases the risk of puncturing the blood vessel during vascular intervention surgery. How to provide more degrees of freedom of motion for vascular intervention navigation surgery systems, effectively detect the resistance of guidewires in the blood vessel to avoid puncturing the blood vessel and provide other functions, so as to carry out more complex vascular intervention surgeries has not been well solved.

Therefore, there is still a lack of a motion system in this field that can provide more degrees of freedom of motion, effectively detect the resistance of the guidewire in the blood vessel, avoid puncturing the blood vessel, and provide other functions for vascular intervention navigation surgery.

### Summary of the invention

The purpose of the present invention is to provide a motion system for a vascular intervention navigation surgery system. The motion system of the present invention provides more functions for the vascular intervention navigation surgery system overall, catheter, guidewire, stent, etc. by adding a support base, Y-connector rotation control structure, guidewire limiting structure, etc., making it more convenient to carry out the vascular intervention surgery. The motion system of the present invention further comprises a force reproduction system for the vascular intervention navigation surgery system. The force reproduction system of the present invention can effectively detect a resistance of the guidewire in the blood vessel, and by processing the resistance, determine the further movement status of the guidewire, thereby improving the safety and operational efficiency of the vascular intervention surgery.

The present invention provides a motion system for a vascular intervention navigation surgery system, wherein the motion system is configured to control the overall motion of the vascular intervention navigation surgery system. The motion system comprises a support base which comprises a support frame and a base, wherein the base provides support for the support frame, the vascular intervention navigation surgery system is configured to clamp a medical device, and the medical device comprises a balloon catheter, a guidewire and a stent, the vascular intervention navigation surgery system is slidable relative to the support base.

In another preferred example, a movable slider is provided on the support frame, and a connecting female head is fixedly installed on the slider; and bottom of the vascular intervention navigation surgery system is fixedly installed with a connecting male head for connection with the connecting female head.

In another preferred example, an electric motor is installed inside the support frame, and the electric motor drives the slider to move in a straight line on the support frame; after the connecting female head on the slider is paired and connected with the connecting male head at the bottom of the vascular intervention navigation surgery system, the vascular intervention navigation surgery system moves in a straight line with the slider on the support frame.

Optionally, the top of the support frame is equipped with an induction device, which is configured to sense the position and distance of the connecting male head and/or the connecting female head.

In another preferred example, a top of the support frame is equipped with a gear set in which a pair of gears meshes with each other, the gear set includes a driving wheel and a passive wheel, the driving wheel is driven by an electric motor, and a bottom of the vascular intervention navigation surgery system is equipped with a rack that matches the gear set; and the rack is driven to move by driving the driving wheel to rotate, thereby causing the vascular intervention navigation surgery system to move.

In another preferred example, a top of the support frame is equipped with a single gear, and a bottom of the vascular intervention navigation surgery system is equipped with an unilateral rack (or single-side rack) that is matched with the single gear; by driving the single gear to rotate, the unilateral rack is driven to move, thereby causing the vascular intervention navigation surgery system to move.

In another preferred example, a top of the support frame is equipped with a longitudinal gear, and a bottom of the vascular intervention navigation surgery system is equipped with a longitudinal rack that matches the longitudinal gear; by driving the longitudinal gear to rotate, the longitudinal rack is driven to move, thereby causing the vascular intervention navigation surgery system to move.

In another preferred example, a top of the support frame is equipped with a transverse gear, a support platform is provided above the support frame, a bottom of the support platform is equipped with an unilateral transverse rack that is matched with the transverse gear, a top of the support platform is equipped with a magnetic suction device, and a bottom of the vascular intervention navigation intervention surgery system is equipped with a magnetic structure, which is configured to attract magnetically and tightly with the support platform; and the unilateral transverse rack is driven to move by driving the transverse gear to rotate, thereby causing the vascular intervention navigation surgery system to move.

In another preferred example, the base comprises a C-shaped groove and a connecting rod; the connecting rod runs through the C-shaped slot, and the C-shaped slot can move up and down along the connecting rod to adjust height; the connecting rod is connected to the support frame through a hinge structure, and the support frame can rotate around the connecting rod to adjust angle.

In another preferred example, the C-shaped slot can be fixed on the surgical bed.

In another preferred example, the vascular intervention navigation surgery system comprises a Y-connector platform, which is configured to accommodate and fix a Y-connector and a guiding catheter connected to the Y-connector;
a distal end of the Y-connector is equipped with a wheel sleeve, which is fixedly connected coaxially with a main channel of the Y-connector, the wheel sleeve is driven by a wheel sleeve driving mechanism to rotate, thereby driving the guiding catheter to rotate.

In another preferred example, the vascular intervention navigation surgery system comprises a guidewire limiting structure, which is set between a fixed plate and the Y-connector platform; the guidewire limiting structure comprises a positioning groove and a cover plate, wherein the positioning groove is configured to accommodate the guidewire, and the positioning groove corresponds to a wire groove of the fixed plate, jointly forming a guidewire channel for placing the guidewire; the cover plate is equipped with one or more protruding cover pieces, which can be inserted into the positioning groove so as to cover and reduce an opening area of the positioning groove, thereby limiting the guidewire.

A motion system for a vascular intervention navigation surgery system, wherein the motion system is configured to control the overall motion of the vascular intervention navigation surgery system, and the motion system comprises: a supporting base, comprising a supporting frame and a base, wherein the supporting frame has a slider that can move left and right, and a connecting female head is fixedly installed on the slider; and a vascular intervention navigation surgery system for clamping medical instruments used in vascular intervention navigation surgery, such as guidewires, catheters, stents, etc. and bottom of the vascular intervention navigation surgery system is fixedly installed with a connecting male head for connection with the connecting female head on the support frame.

In another preferred example, the support frame is internally equipped with a electric motor. The electric motor drives the slider to move left and right in a straight line on the support frame. After the connecting female head on the slider is paired and connected with the connecting male head at the bottom of the vascular intervention navigation surgery system, the vascular intervention navigation surgery system can move left and right in a straight line on the support frame.

In another preferred example, the slider is fixedly connected to the connecting female head through clamping, welding, bonding, integrated molding, and other methods.

In another preferred example, the vascular intervention navigation system is fixedly connected to the connecting male through methods such as clamping, welding, bonding, and integrated molding.

In another preferred example, the supporting base is matched with the vascular intervention navigation system through the connecting female head and the connecting male head, and is fixed together through thread engagement and/or buckle connection.

In another preferred example, the base is composed of a C-shaped slot and a connecting rod. The base is composed of a C-shaped slot and a connecting rod, which is configured to fix the motion system next to the surgical bed.

In another preferred example, the connecting rod runs through the C-shaped slot, which can move up and down on the connecting rod to adjust the height.

In another preferred example, the connecting rod is connected to the support frame through a hinge structure, and the support frame can rotate on the connecting rod to adjust the angle.

In another preferred example, there is an induction device at the top of the support frame. The induction device can sense the position and distance of the male (female) head connected to it.

In another preferred example, the induction device may be laser, infrared, or mechanical.

In another preferred example, the vascular intervention navigation surgery system comprises a Y-connector platform, which can place and fix the Y-connector and the guiding catheter connected to it. The front end of the Y-connector has a wheel sleeve, which is fixedly connected coaxially with the main channel of the Y-connector. The wheel sleeve is driven by a wheel sleeve driving mechanism to rotate, thereby driving the guiding catheter to rotate.

In another preferred example, the wheel sleeve is fixed to the front end of the Y-connector through a polygonal inner hole.

In another preferred example, the wheel sleeve is fixedly connected to the Y-connector through clamping, welding, bonding, integrated molding, and other methods.

In another preferred example, the wheel sleeve is a gear or worm gear.

In another preferred example, the wheel sleeve driving mechanism is a gear, rack, worm, etc.

In another preferred example, the vascular intervention navigation surgery system comprises a guidewire limiting structure, which is set between a fixed plate and the Y-connector platform. The guidewire limiting structure comprises a positioning groove and a cover plate, wherein the positioning groove is configured to accommodate the guidewire, and the positioning groove corresponds to a wire groove of the fixed plate, jointly forming a guidewire channel for placing the guidewire; The cover plate is equipped with one or more protruding cover pieces, which can be inserted into the positioning groove, covering the opening area of the positioning groove, and thereby limiting the guidewire.

In another preferred example, there is a pair of gears meshing with each other at the top of the support frame, which is composed of a driving wheel and a passive wheel. The driving wheel is driven by an electric motor, and the bottom of the vascular intervention navigation surgery system has a rack that matches the gear set. By driving the driving wheel of the gear set to rotate, the rack is driven to move, thereby causing the vascular intervention navigation surgery system to move.

In another preferred example, the top of the support frame has a single gear, and the bottom of the vascular intervention navigation surgery system has a unilateral rack that is matched with the single gear. By driving the rotation of the single gear, it drives the movement of the unilateral rack, thereby causing the vascular intervention navigation surgery system to move.

In another preferred example, the top of the support frame has a longitudinal gear, and the bottom of the vascular intervention navigation surgery system has a longitudinal gear that matches the longitudinal gear. By driving the longitudinal gear to rotate, it drives the longitudinal gear to move, thereby causing the vascular intervention navigation surgery system to move.

In another preferred example, the top of the support frame has a transverse gear, and there is a support platform above the support frame. The bottom of the support platform has a single-sided transverse rack that matches the transverse gear. The top of the support platform has a magnetic suction device, and the bottom of the vascular intervention navigation surgery system has a magnetic structure that attract magnetically and tightly with the support platform. By driving the transverse gear to rotate, the single-sided transverse rack moves, thereby causing the vascular intervention navigation surgery system to move.

In another preferred example, the motion system comprises a host, through which motion parameters of the motion system are controlled.

In another preferred example, the motion system comprises a handle, which is signal connected to the host (such as via WiFi, 5G, Bluetooth, etc.), and the host can be remotely controlled through the handle.

In another preferred example, the support base is equipped with a control screen, by which the movement of the vascular intervention navigation surgery system is controled.

A force reproduction system for a vascular intervention navigation surgery system, which comprises a force reproduction system adjunct, and the force reproduction system adjunct comprises: a guidewire fixator configured to clamp and fix a guidewire; a pressure sensor comprising a pressure sensing plate, and a sliding sleeve component comprising a first sliding kit and a second sliding kit, wherein one end of the first sliding kit is fixedly connected to the guidewire fixator, and the first sliding kit can slide relative to the second sliding kit so that the other end of the first sliding kit can selectively press against the pressure sensing plate.

In another preferred example, the vascular intervention navigation surgery system comprises a guidewire control module for controlling forward, backward, and rotation of the guidewire, and the guidewire control module comprises: a rotating assembly configured to control the rotation of the guidewire, including rotating wheel set, a rotating shaft concentrically connected to the rotating wheel set, a planet gear sleeved on the rotating shaft and slidable relative to the rotating shaft, and a sun gear meshing with the planet gear, wherein the sun gear is provided with a wire slot, which opens from a valley between teeth of the sun gear to center of the sun gear, and is configured to insert the guidewire and ensure the coaxial rotation of the guidewire; and a traveling component configured to control the advance or retreat of the guidewire, including a traveling wheel set, a transmission screw concentrically connected with a bevel gear of the traveling wheel set, and a fixed disc for supporting the sun gear, which is threaded and connected to the transmission screw rod.

In another preferred example, the force reproduction system adjunct is fixed to the sun wheel, moving forward, backward, and rotating together with the sun wheel.

In another preferred example, the force reproduction system adjunct advances and rotates together with the guidewire.

In another preferred example, the force reproduction system adjunct comprises an electric induction magnetic coil, wherein the electric induction magnetic coil is attracted to the guidewire fixator and/or the first sliding kit.

In another preferred example, the sliding sleeve component is coaxial with the pressure sensor.

In another preferred example, the guidewire fixator and the sliding sleeve component are eccentrically set.

In another preferred example, the guidewire fixator is a sleeve which is a hollow tube that runs through the front and back, and the guidewire passes through the sleeve and is clamped and fixed by the inner wall of the sleeve.

In another preferred example, the inner wall of the sleeve is a rubber surface, and the guidewire is clamped by the inner wall through friction.

In another preferred example, the force reproduction system further comprises a force reproduction system main end, wherein the force reproduction system main end comprises a wheel group and a simulated guidewire placed between wheels of the wheel group, wherein the resistance of the wheel group to the simulated guidewire is equivalent to the resistance experienced by the guidewire during the forward process. That is to say, the resistance of the wheel group to the simulated guidewire is equal to the force value sensed by the pressure sensor.

In another preferred example, when the operator feels the resistance of the simulated guidewire, they rely on their own experience to determine whether to continue operating the sun wheel to control the guidewire to move forward or rotate.

In another preferred example, the rear end of the wheel group is connected to a electric motor, which controls the pressure between the wheels of the wheel group through the rotation of the electric motor, i.e. controls the resistance of the simulated guidewire between the wheels of the wheel group.

In another preferred example, when the electric motor rotates clockwise or counterclockwise, the pressure between the wheels of the wheel group may increase or decrease, and the resistance of the simulated guidewire between the wheels of the wheel group may increase or decrease.

In another preferred example, the force reproduction system main end includes a simulated pressure sensor, which is configured to sense the operator's thrust on the simulated guidewire, so that the thrust is further applied on the guidewire.

In another preferred example, when the operator pushes the simulated guidewire against the resistance of the wheel group, the motion of the simulated guidewire drives the rotation of the wheel group, and controls the forward or backward movement of the sun wheel group based on the rotation of the wheel group sensed by the simulated pressure sensor, thereby driving the forward or backward movement of the guidewire.

In another preferred example, the conversion from the rotational motion of the wheel group to the forward or backward rotational angle of the sun wheel group to the linear distance is processed by the main end control system.

In another preferred example, the simulated guidewire is supported by a guidewire tray.

In another preferred example, the simulated guidewire is connected end-to-end and is cyclically pushed.

In another preferred example, the guidewire tray may be, but not limited to, circular, round, or arc-shaped.

In another preferred example, the guidewire tray has a guidewire groove, and the simulated guidewire is embedded in the guidewire groove; The guidewire tray is equipped with a guidewire bridge. The simulated guidewire detaches from the guidewire groove at the guidewire bridge, and the operator grasps and pushes the simulated guidewire at the guidewire bridge.

In another preferred example, the length of the guidewire bridge is 1-20 centimeters; Preferred 3-6 centimeters.

In another preferred example, the diameter of the simulated guidewire is 0.1-0.7 millimeters; Preferably, 0.2-0.5 millimeters; Better yet, 0.3-0.4 millimeters; Best of all, 0.35 millimeters.

A control method for a force reproduction system for a vascular intervention navigation surgery system, comprising:
(a) Provide a force reproduction system for vascular intervention navigation surgery system as described above;
(b) When the force reproduction system main end simulates the resistance of the guidewire, the operator pushes the simulated guidewire at the force reproduction system main end to overcome the simulated resistance, and the generated pushing force value is transmitted to the main control system. The main control system sends a forward command to the sun gear group, and the forward generated pushing force value is equal to the operator's pushing force at the force reproduction system main end.

The main advantages of the present invention comprise:
(a) The vascular intervention navigation surgery system has increased its travel time, adapting to more surgical scenarios;
(b) The vascular intervention navigation surgery system has increased functionality, making it easier to perform more complex surgeries;
(c) Compact in size, does not occupy limited operating room space;
(d) Automated control, more precise surgical control;
(e) The force reproduction system can effectively detect the resistance of the guidewire in the blood vessel, and by processing the resistance, the further movement status of the guidewire is determined, which improves the safety and operational efficiency of vascular intervention surgery.

It should be understood that within the scope of the present invention, the above-mentioned technical features of the present invention and the technical features specifically described in the following (such as Examples) can be combined with each other to form a new or preferred technical solution. Due to the length of the article limitation, they will not be repeated here.

### Description of the drawings

In order to illustrate the technical solutions in the embodiments of the present invention or in the prior art more clearly, the drawings required for the description of the embodiments or the prior art will be briefly described below. Obviously, the drawings in the following description are only some embodiments of the present invention, and those of ordinary skill in the art may obtain other drawings according to these drawings without creative efforts.
Figure 1 is a perspective view of the motion system of a vascular intervention navigation surgery system according to an example of the present invention;
Figure 2 is a perspective view of a supporting base according to an example of the present invention;
Figure 3 is a perspective view of a vascular intervention navigation surgery system according to an example of the present invention;
Figure 4 is a bottom view of Figure 3;
Figure 5A is a rear view of Figure 2;
Figure 5B is a perspective view of a variant of the base of the motion system of a vascular intervention navigation surgery system according to an example of the present invention;
Figure 6 is a top view of Figure 3;
Figure 7 is a perspective view of a motion system according to other example of the present invention;
Figure 8 is a perspective view of a supporting base according to other example of the present invention;
Figure 9 is a perspective view of a supporting base according to another example of the present invention;
Figure 10 is a bottom view of a vascular intervention navigation surgery system according to another example of the present invention;
Figure 11 is a perspective view of a supporting base according to yet another example of the present invention;
Figure 12 is a bottom view of a vascular intervention navigation surgery system according to yet another example of the present invention;
Figure 13 is a perspective view of a motion system according yet another example of the present invention;
Figure 14 is a perspective view of a supporting base according to yet another example of the present invention;
Figure 15 is a bottom view of a vascular intervention navigation surgery system according to yet another example of the present invention;
Figure 16 is a perspective view of a force reproduction system adjunct according to an example of the present invention;
Figure 17 is a side view of the force reproduction system adjunct in Figure 16;
Figure 18 is a cross-sectional view taken along the A - A section of the force reproduction system adjunct in Figure 17;
Figure 19 is a perspective view of the force reproduction system adjunct in the form of a guidewire sleeve for a guidewire fixator;
Figure 20 is an exploded view of the force reproduction system adjunct in Figure 19;
Figure 21 is a perspective view of the force reproduction system main end according to an example of the present invention;
Figure 22 is perspective view of the force reproduction system main end in Figure 21, with the top cover removed.

In the figures, references are as follows:
1- Supporting base;
2- Vascular interventional navigation surgery system;
3- Slider;
4- Connecting female head;
5- Induction device;
6- Y-connector platform;
7- Y-connector;
8- Wheel sleeve;
9- Fixed plate;
10- Cover plate;
11- Positioning groove;
12- Cover piece;
13- Connecting male head;
14- C-shaped slot;
15- Connecting rod;
16- Gear set;
17- Single gear;
18- Unilateral rack;
19- Longitudinal gear;
20- Longitudinal rack;
21- Supporting platform;
22- Magnetic structure;
23- Sun gear;
24- Fixed disc;
25- Guidewire fixator;
26- Pressure sensing plate;
27- First sliding kit;
28- Second sliding kit;
29- Connecting base;
30- Force reproduction system main end;
31- Wheel group;
32- Simulated guidewire;
33- Guidewire tray;
34- Guidewire groove;
35- Guidewire bridge;
36- First arm;
37- Second arm;
38- Slot;
39- Insertion section.

### Detailed Description

After extensive and in-depth research, the inventor has developed a motion system for a vascular intervention navigation surgery system for the first time through extensive screening. The motion system of the present invention provides more degrees of freedom of motion and other functions for the vascular intervention navigation surgery system overall, guidewire, catheter, stent, etc. by adding a support base, Y-connector rotation control structure, guidewire limiting structure, etc., making it more convenient to carry out vascular intervention surgery; The motion system of the present invention further includes a force reproduction system for a vascular intervention navigation surgery system; The force reproduction system of the present invention can effectively detect the resistance of the guidewire in the blood vessel. By processing the resistance, the further movement status of the guidewire is determined, which improves the safety and operational efficiency of vascular intervention surgery. On this basis, the present invention is completed.

The motion system for a vascular intervention navigation surgery system of the present invention provides greater freedom of motion for the terminal execution system, guide catheter, guidewire, etc. by adding elevation components, movement components, rotation components, Y-connector rotation control parts, guidewire limiting structures, etc., to facilitate the implementation of vascular intervention navigation surgery.

Typically, the present invention provides a motion system for a vascular intervention navigation surgery system, wherein the motion system is configured to control the movement of components within the vascular intervention navigation surgery system. The motion system comprises a terminal execution system motion control portion, wherein the terminal execution system motion control portion comprises a pitch component, wherein the pitch component is a hinge structure, and the pitch component comprises a first connector (such as a support arm) and a first rotating member (such as a tray), wherein the first rotating member can rotate around the first connector; And a mobile component, wherein the mobile component is a linear motion structure, the mobile component comprises a first moving member connected to the first rotating member (such as a support frame) and a second moving member that can slide relative to the first moving member (such as a second connecting plate of the terminal execution system).

In another preferred example, the terminal execution system motion control portion comprises a rotating component, wherein the rotating component comprises a second connector (such as a C-shaped slot), and the first connector is rotatable around the second connector.

In another preferred example, the second connector is fixed.

In another preferred example, the second connector is fixed on the hospital bed, for example, by clamping or fixed on the ground.

In another preferred example, the first rotating member and the first moving member are fixedly connected.

In another preferred example, the first moving member is rotatable relative to the first rotating member.

In another preferred example, the first connector can be retracted or moved up and down.

In another preferred example, the second moving member is fixedly connected to the terminal execution system.

In another preferred example, the second moving member is fixedly connected to the terminal execution system through clamping, welding, bonding, integrated molding, and other methods.

In another preferred example, the first moving member and the second moving member move relative to each other through belt/chain transmission, gear rack transmission, screw transmission, and other means.

In another preferred example, the first moving member is equipped with a first connecting plate, and the second moving member is equipped with a second connecting plate. The first connecting plate and the second connecting plate are adapted, and the two are fixedly connected through clamping, welding, bonding, and other methods. The first moving member has a driving device, which is configured to control the first connecting plate, Furthermore, it controls the movement of the second connecting plate and ultimately drives the terminal execution system.

In another preferred example, the second moving member is equipped with a hollow cylindrical slot as the second connecting plate, and the inner side of the slot has a threaded structure. The first moving member is equipped with a protrusion that matches the slot and the threaded structure as the first connecting plate.

In another preferred example, the first connecting plate and the second connecting plate are fixed together through thread engagement and/or snap fit.

In another preferred example, the first moving member is a transverse gear, and the second moving member is a transverse rack which is matched with the transverse gear and is set on the terminal execution system. By driving the transverse gear to rotate, it drives the transverse rack to move, thereby causing the terminal execution system to move; or

The first moving member is a vertical gear, and the second moving member is a vertical rack that is matched with the vertical gear. By driving the vertical gear to rotate, it drives the vertical rack to move, thereby causing the terminal execution system to move.

The number of mutually matched gears and racks mentioned above can be one pair, two pairs, or more pairs, which can be arranged according to actual operational needs.

In another preferred example, the motion system comprises a Y-connector rotation control portion, wherein the Y-connector rotation control portion comprises a wheel sleeve and a wheel sleeve driving mechanism, wherein the wheel sleeve is fixedly connected coaxially to the main channel of the Y-connector, and the wheel sleeve drives the rotation through the wheel sleeve driving mechanism, thereby driving the Y-connector to rotate.

In another preferred example, the wheel sleeve is located on the outer circumference of the Y-connector.

In another preferred example, the wheel sleeve and Y-connector are integrated structures.

In another preferred example, the gear sleeve is a gear or worm gear.

In another preferred example, the wheel sleeve driving mechanism is a gear, rack, worm, etc.

In another preferred example, the terminal execution system comprises a guidewire limiting structure, which is set between the fixed plate and the Y-connector to prevent the guidewire from arching.

In another preferred example, the guidewire limiting structure comprises a positioning groove and a cover plate, wherein the positioning groove is configured to accommodate the guidewire, and the positioning groove corresponds to the wire groove on the sun wheel and the wire groove on the fixed plate, jointly forming a guidewire channel for placing the guidewire; The cover plate is equipped with one or more protruding cover pieces, which can be inserted into the positioning groove, covering the opening area of the positioning groove, and thereby limiting the guidewire.

In another preferred example, a locator is provided on the first moving member, and an induction block is provided on the second moving member. The locator can sense the movement of the induction block to determine the movement distance of the terminal execution system.

In another preferred example, at startup, the locator senses the induction block and places the terminal execution system in the zero position.

In another preferred example, the locator may be laser, infrared, or mechanical.

In another preferred example, the motion system comprises a host, through which the motion parameters of the motion system are controlled.

In another preferred example, the motion system comprises a handle, which is signal connected to the host (such as via Wi-Fi, 5G, Bluetooth, etc.), and the host can be remotely controlled through the handle.

In another preferred example, the first moving member is equipped with a control screen, by which the sliding of the second sliding member relative to the first sliding member is controled.

Specifically, the motion system for the vascular intervention navigation surgery system of the present invention is configured to control the movement of components within the vascular intervention navigation surgery system, including a terminal execution system motion control portion.

The terminal execution system motion control portion includes a pitch component, a rotation component, a movement component, and a height adjustment component. The pitch component is a hinge structure, which includes a first connecting member (shown as connecting rod 8) and a first rotating member (such as a cantilever, shown as the lower part of the supporting base 1), wherein the first rotating member can rotate around the first connecting member. The moving component is a linear motion structure, which includes a first moving member connected to the first rotating member (shown as the upper part of the supporting base 1) and a second moving member that can slide relative to the first moving member (such as a connecting female head 4 or a component including a connecting female head 4). The rotating component includes a first rotating member and a first moving member, that is, the first moving member can rotate relative to the first rotating member. The height adjustment component includes a second connector (shown as C-shaped slot 14), a first connector, and a height adjustment pin. The second connector can be fixed to the hospital bed, for example by clamping or fixed to the ground to provide support. The position relationship between the first connector and the second connector can be fixed by the height adjustment pin. The first connector is equipped with height adjustment holes (not shown) that are compatible with the height adjustment pin. By inserting the height adjustment pin into different height adjustment holes, the height of the terminal execution system can be adjusted.

The first moving member is equipped with a control screen, which controls the sliding of the second sliding member relative to the first sliding member.

The motion system also includes a host and a handle. The motion parameters of the motion system are controlled through the host. The handle is signal connected to the host (such as via Wi-Fi, 5G, Bluetooth, etc.), and the host can be remotely controlled through the handle.

The first and second moving members move relative to each other through belt/chain transmission, gear and rack transmission, screw transmission, and other means. Preferably, the first moving member is equipped with a locator, and the second moving member is equipped with an induction block. The locator can sense the movement of the induction block to determine the movement distance of the terminal execution system. The locator can be laser, infrared, or mechanical. When starting, the locator senses the induction block and places the terminal execution system at zero position.

The second moving member is fixedly connected to the terminal execution system. The second moving member is fixedly connected to the terminal execution system through clamping, welding, bonding, and integrated molding. The first moving member is equipped with a first connecting plate (for example, shown as a connecting male 13). The first connecting plate and the second connecting plate (e.g. shown as the connecting female head 4) are compatible, and the two are fixed and connected by means of clamping, welding, bonding, etc. For example, the second connecting plate can be a hollow cylindrical slot, with a threaded structure on the inner side of the slot. The first connecting plate can have protrusions that match the slot and the threaded structure. The first connecting plate and the second connecting plate are fixed together through thread engagement and/or snap fit. The first moving member has a driving device, which is configured to control the first connecting plate, thereby controlling the movement of the second connecting plate and ultimately driving the terminal execution system.

In another example, the first moving member is a single gear (horizontal gear) 17, and the second moving member is a unilateral rack (vertical rack) 18 that is set on the terminal execution system and matched with the single gear (horizontal gear) 17. By driving the single gear (horizontal gear) 17 to rotate, it drives the unilateral rack (vertical rack) 18 to move, thereby causing the terminal execution system to move.

In another example, the first moving member is a gear set (two transverse gears) 16, and the second moving member is two vertical racks that are respectively matched with the gear set (two transverse gears) 16 set on the terminal execution system. By driving the gear set (two transverse gears) 16 to rotate, the vertical racks are driven to move, thereby causing the terminal execution system to move.

In another example, the first moving member is the longitudinal gear (vertical gear) 19, and the second moving member is the longitudinal rack (horizontal rack) 20 that matches the longitudinal gear (vertical gear) 19. By driving the longitudinal gear (vertical gear) 19 to rotate, it drives the longitudinal rack (horizontal rack) 20 to move, thereby causing the terminal execution system to move.

In one example, there is a horizontal gear at the top of support frame 1, and a support platform 21 above support frame 1. At the bottom of support platform 21, there is a unilateral horizontal rack that matches the horizontal gear. At the top of support platform 21, there is a magnetic suction device. At the bottom of vascular intervention navigation surgery system 2, there is a magnetic structure 22 that is tightly attached to the support platform 21. By driving the horizontal gear to rotate, it drives the unilateral horizontal rack to move, thereby causing the vascular intervention navigation surgery system to move.

The motion system for the vascular intervention navigation surgery system also includes a Y-connector rotation control portion. The Y-connector rotation control portion includes a wheel sleeve 8 and a wheel sleeve driving mechanism (not shown). The wheel sleeve 8 is fixedly connected to the main channel of the Y-connector 7 coaxially, and is set on the outer circumference of the Y-connector 7. The wheel sleeve 8 drives the rotation through the wheel sleeve driving mechanism, thereby driving the Y-connector 7 to rotate. The wheel sleeve 8 can be a gear or a worm gear. The wheel drive mechanism can be gears, racks, worms, etc.

The motion system for the vascular intervention navigation surgery system comprises a guidewire limiting structure. The guidewire limiting structure is set between the fixed plate 9 and the Y-connector 7 to prevent the guidewire from arching. The guidewire limiting structure includes a positioning groove 11 and a cover plate 10. The positioning groove 11 is configured to accommodate the guidewire. The positioning groove 11 corresponds to the wire groove on the sun wheel and the wire groove on the fixed plate 9, jointly forming a guidewire channel for placing the guidewire. The cover plate 10 is equipped with one or more protruding cover pieces 12, which can be inserted into the positioning groove 11, covering the opening area of the positioning groove 11 and thereby limiting the guidewire.

Typically, a force reproduction system for the vascular intervention surgery navigation system of the present invention is used for vascular intervention treatment, including a remote microcomputer control end, a surgical positioning robot arm and a terminal execution system, wherein the terminal execution system is fixed on the end of the surgical positioning robot arm and moves with the surgical positioning robot arm, and the remote microcomputer control end controls the movement of the surgical positioning robot arm and the movement inside the terminal execution system.

The terminal execution system is configured to drive the forward, backward, and rotation of interventional equipment, including a guidewire control module, a balloon/stent control module, and a guide catheter control module.

The force reproduction system adjunct is set on the guidewire control module of the terminal execution system, configured to measure the resistance of the working guidewire when moving forward or backward in the blood vessel.

The force reproduction system adjunct consists of a pressure sensor, a guidewire series connector, a working guidewire, a resistance detection guidewire, and a connecting base. The pressure sensor is fixed on the connecting base, and the connecting base is fixed on the sun wheel of the guidewire control module.

There is a wire groove on the connecting base, and the bottom of the groove is at the same horizontal height as the center of the sun wheel. When the sun gear rotates, the connecting base rotates coaxially with the sun gear, and the wire groove on the connecting base is always aligned with the wire groove of the sun gear.

The guidewire series connector is a U-shaped guidewire series connector consisting of two parallel guidewire sleeves, one end of which is a fixed component that fixes the two parallel sleeves. The parallel sleeves and the fixed component form a U-shaped guidewire series connector.

The working guidewire sleeve is a through sleeve, where the working guidewire enters from the front end of the sleeve and exits from the back end. It is embedded in the wire groove of the connecting base at the connecting base and embedded in the wire groove of the sun gear at the sun gear. The working guidewire is fixed after passing through the center of the sun gear. The length range of the working guidewire sleeve is 1-100 millimeters.

The front end of the resistance detection guidewire sleeve is sealed by a fixing component, and a resistance detection guidewire is inserted into the rear end of the sleeve.

One end of the resistance detection guidewire is fixed in the resistance detection guidewire sleeve, and the other end is inserted into the resistance detection guidewire fixed tube on the pressure sensor. The top of the guidewire is just in contact with the contact surface of the pressure sensor. The length range of the resistance detection sleeve is 1-100 millimeters.

Alternatively, the working guidewire and resistance detection guidewire can be fixed with a guidewire fixing clip. The structure of the guidewire fixing clip is two clips connected at the tail, with the upper clip fixing the working guidewire and the lower clip fixing the resistance detection guidewire.

On the outer ring of the pressure sensor, an electric induction magnetic coil is installed. After being energized, the magnetic coil generates magnetism and attracts the resistance detection guidewire to fix the tube. When the pressure sensor moves forward and backward, it will move forward and backward together with the resistance detection guidewire and fixed tube.

The remote microcomputer control end has a force reproduction system main end. When the pressure sensor senses the resistance value, the force reproduction system main end needs to reproduce an equivalent simulated force. The doctor pushes the simulated guidewire of the force reproduction system main end outside the operating room to determine the working guidewire's forward and backward movement in the real blood vessel. The force reproduction system main end consists of a simulated guidewire, a wheel group, a push rod, and a pressure sensor. The wheel group consists of two independent wheels, with the wheel surfaces in contact with each other. The simulated guidewire passes between the two wheels, and the wheels on both sides compress the simulated guidewire. The extrusion force is the resistance of the simulated guidewire to move forward and backward. The forward and backward movement of the push rod can adjust the distance between two wheels. When the push rod is pushed forward, the gap between the two wheels decreases and the friction force increases. When the push rod is pushed backward, the gap between the two wheels increases and the friction force decreases. One of the wheels is equipped with a pressure sensor on the wheel surface. When the two wheels come into contact, the pressure sensor senses the pressure and displays the pressure value.

When the guidewire module advances, the connecting base fixed on the sun wheel pushes the pressure sensor, the resistance detection guidewire, and the guidewire series connector forward together. At this point, the reading on the pressure sensor is the thrust of the working guidewire to move forward.

When the front end of the working guidewire is obstructed from moving forward, and the resistance to it increases, the guidewire serial connector transfers the resistance to the pressure sensor through the resistance detection guidewire. The reading on the pressure sensor is the thrust of the working guidewire to move forward and the resistance to the working guidewire.

At the initial stage of the force reproduction system, there is no pressure between the wheels of the wheel group. When the simulated guidewire is pushed, it drives the wheel group to rotate, and the generated signal is transmitted to the main control system. The main control system sends instructions to the sun wheel group to move forward or backward, thereby driving the guidewire to move forward or backward.

The simulated guidewire diameter used at the force reproduction system main end is 0.35 millimeters. After passing through the wheel group, the guidewire is placed on the guidewire tray, which has a guidewire groove to connect the simulated guidewire head and tail and repeat the push. The guidewire tray is circular, can also be round or arc-shaped.

There is a gap on the guidewire tray for the surgeon to pinch, push, pull, or twist the simulated guidewire with their fingers. The length of the gap is 1-20 centimeters, preferably 3-6 centimeters.

The present invention is further described below in conjunction with specific embodiments. It should be understood that these examples are intended to illustrate the invention only and not to limit the scope of the invention. Furthermore, the drawings are schematic diagrams, and therefore the apparatus and device of the present invention are not limited by the size or scale of the schematic diagrams.

It should be noted that in the claims and specification of the present application, relational terms such as first and second, etc. are only used to distinguish one entity or operation from another entity or operation, and do not necessarily require or imply that there is any such actual relationship or order between these entities or operations. Moreover, the terms "comprise", "include" or any other variant thereof are intended to cover non-exclusive inclusions such that a process, method, article or device comprising a series of elements comprises not only those elements, but also other elements not explicitly listed, or also elements inherent to such process, method, article or device. In the absence of further restrictions, the element defined by the statement "include(s) a/an" do not exclude the existence of other identical elements in the process, method, article or device including the element.

### Example

The motion system for the vascular intervention navigation surgery system in this Example is used to control the motion of the vascular intervention navigation surgery system.

The vascular intervention navigation surgery system comprises a guidewire control module for controlling forward, backward, and rotation of the guidewire, and the guidewire control module comprises: a rotating assembly configured to control the rotation of the guidewire, including rotating wheel set, a rotating shaft concentrically connected to the rotating wheel set, a planet gear sleeved on the rotating shaft and slidable relative to the rotating shaft, and a sun gear meshing with the planet gear, wherein the sun gear is provided with a wire slot, which is opened from the bottom of the valley between the teeth of the sun gear to the center of the circle of the sun gear, and the wire groove is configured to insert the guide wire and ensure the coaxiality of the guide wire drive; and a traveling component configured to control the advance or retreat of the guidewire, including a traveling wheel set, a transmission screw concentrically connected with a bevel gear of the traveling wheel set, and a fixed disc 24 for supporting the sun gear, which is threaded and connected to the transmission screw rod.

The vascular intervention navigation surgery system can be the vascular intervention navigation surgery system disclosed in CN 113598947 B, which is incorporated into this article by reference as a whole.

### Example 1

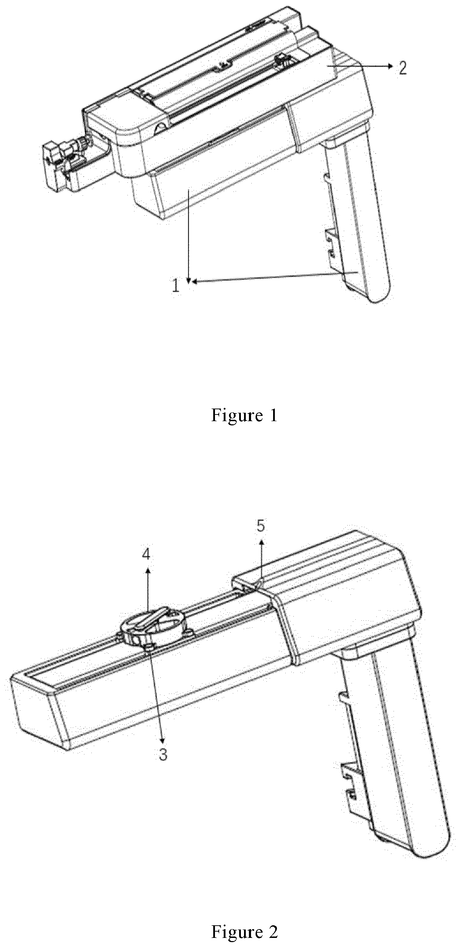

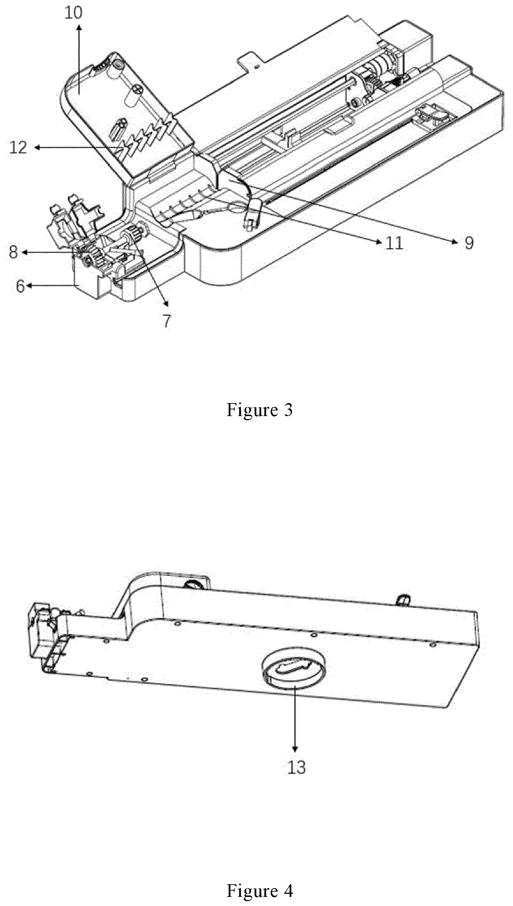

As shown in Figures 1-4, the motion system for the vascular intervention navigation surgery system includes a support base and a vascular intervention navigation surgery system 2. The supporting base includes a supporting frame 1 and a base, and the base includes a C-shaped slot 14 and a connecting rod 15. There is a slider 3 on the support frame 1 that can move left and right. The slider 3 is fixedly connected to the connecting female head 4 through clamping, welding, bonding, and integrated molding. The bottom of the vascular intervention navigation surgery system 2 is fixedly connected to the connecting male head 13 through clamping, welding, bonding, and integrated molding. The interior of the support frame 1 is equipped with an electric motor and a transmission structure, and the electric motor drives the slider 3 to move in a straight line on the support frame 1. The connecting female head 4 on the slider 3 is connected to the connecting male head 13 at the bottom of the vascular intervention navigation surgery system 2 through thread engagement and/or snap fit. The vascular intervention navigation surgery system 2 can move left and right in a straight line on the support frame 1.

There is an induction device 5 on the top of support frame 1, which can sense the position and distance of the male (female) head 13. The induction device 5 can be laser, infrared, or mechanical.

The vascular intervention navigation surgery system 2 includes a Y-connector platform 6, which can place and fix the Y-connector 7 and the guiding catheter connected to it. The front end of the Y-connector 7 is equipped with a wheel sleeve 8, which is fixedly connected coaxially with the main channel of the Y-connector 7. The wheel sleeve 8 is driven to rotate by the wheel sleeve driving mechanism, thereby driving the guiding catheter to rotate.

The vascular intervention navigation surgery system 2 includes a guidewire limiting structure, which is set between the fixed plate 9 and the Y-connector platform 6. The guidewire limiting structure includes a positioning groove 11 and a cover plate 10. The positioning groove 11 is configured to accommodate the guidewire, and the positioning groove 11 corresponds to the wire groove of the fixed plate 9, jointly forming a guidewire channel for placing the guidewire; The cover plate 10 is equipped with one or more protruding cover pieces 12, which can be inserted into the positioning groove 11, covering the opening area of the positioning groove 11 and thereby limiting the guidewire.

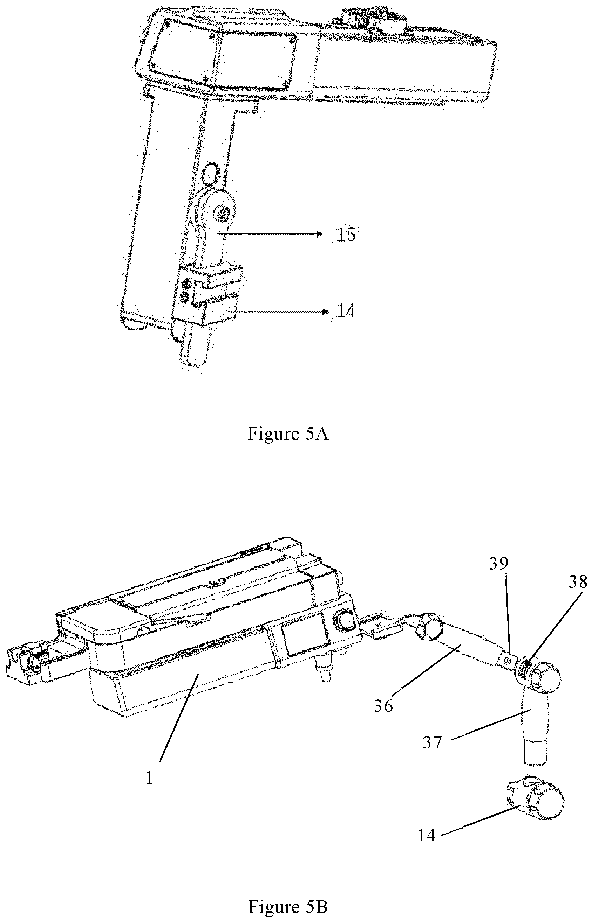

As shown in Figure 5A, the supporting base includes a base, which is composed of a C-shaped slot 14 and a connecting rod 15, to fix the motion system of the vascular intervention navigation surgery system next to the surgical bed. The connecting rod 15 runs through the C-shaped slot 14, which can move up and down on the connecting rod 15 to adjust the height. The connecting rod 15 is connected to the support frame 1 through a hinge structure, and the support frame 1 can rotate and adjust the angle on the connecting rod 15.

In another implementation, as shown in Figure 5B, the base comprises a first support arm 36 and a second support arm 37. The first support arm 36 and the supporting frame 1 are assembled together by clamping, and the second support arm 37 can be inserted into the C-shaped slot 14 for fixation. The second arm 37 is equipped with a slot 38, and the first arm 36 is equipped with an insertion portion 39. By inserting the insertion portion 39 into the slot 38, the fixation between the first arm 36 and the second arm 37 is achieved. Wherein, the second arm 37 can be equipped with multiple slots 38 (shown as three in Figure 5B), and the openings of the multiple slots 38 are facing in different directions. By inserting the insertion portion 39 of the first arm 36 into different slots 38, the angle between the first arm 36 and the second arm 37 can be adjusted, thereby achieving the adjustment of the position and angle of the vascular intervention navigation surgery system. It should be noted that slot 38 can be set on the first arm 36, while the insertion portion 39 can be set on the second arm 37, as long as the angle adjustment between the first arm 36 and the second arm 37 can be achieved.

The motion system also includes a host and a handle. The motion parameters of the motion system are controlled through the host. The handle is connected to the main body signal (such as Wi-Fi, 5G, Bluetooth, etc.), and the host can be remotely controlled through the handle.

### Example 2

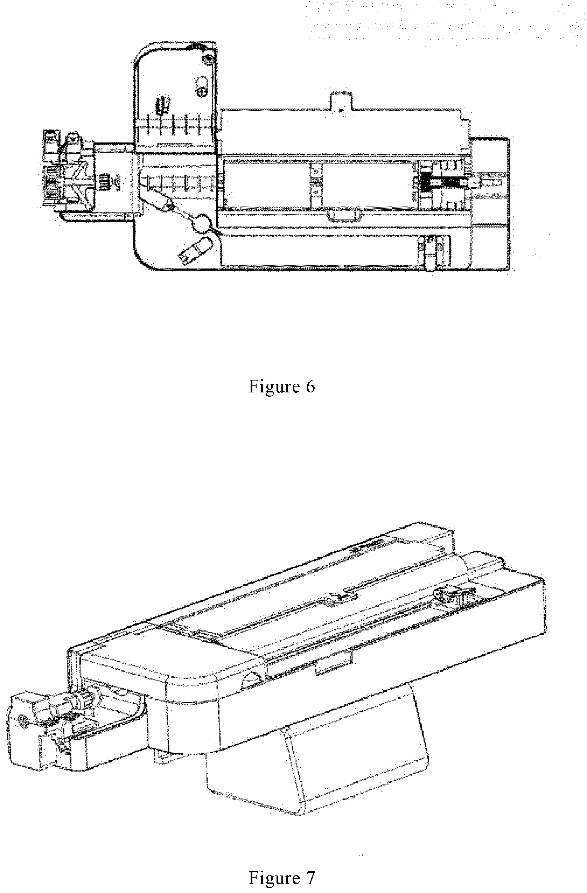

As shown in Figures 7-8, the support frame 1 of the motion system of the vascular intervention navigation surgery system has a pair of meshing gear sets 16 at the top. The gear set 16 is composed of an active wheel and a passive wheel, which are driven by an electric motor. The bottom of the vascular intervention navigation surgery system 2 has a gear rack that matches the gear set 16. By driving the active wheel of the gear set 16 to rotate, the gear rack moves, causing the vascular intervention navigation surgery system 2 to move.

### Example 3

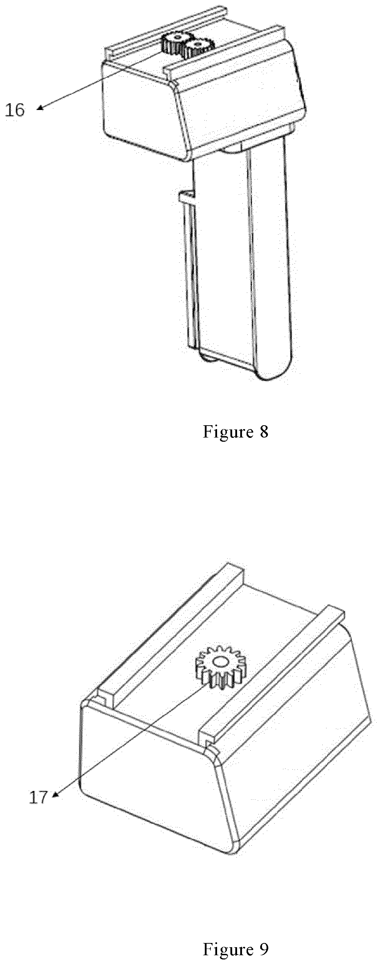

As shown in Figures 9-10, the support frame 1 of the motion system for the vascular intervention navigation surgery system has a single gear 17 at the top, and the bottom of the vascular intervention navigation surgery system 2 has a unilateral rack 18 that is matched with the single gear 17. By driving the single gear 17 to rotate, it drives the unilateral rack 18 to move, thereby causing the vascular intervention navigation surgery system 2 to move.

### Example 4

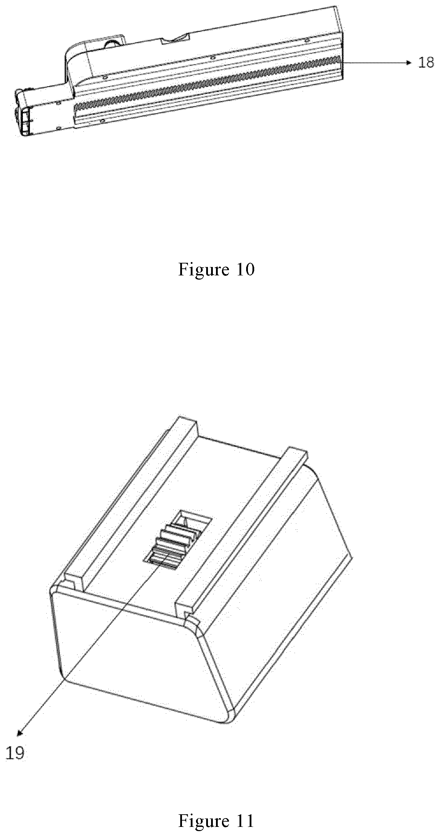

As shown in Figures 11-12, the support frame 1 of the motion system of the vascular intervention navigation surgery system has a longitudinal gear 19 at the top, and the bottom of the vascular intervention navigation surgery system 2 has a longitudinal rack 20 that matches the longitudinal gear 19. By driving the longitudinal gear 19 to rotate, it drives the longitudinal rack 20 to move, thereby causing the vascular intervention navigation surgery system 2 to move.

### Example 5

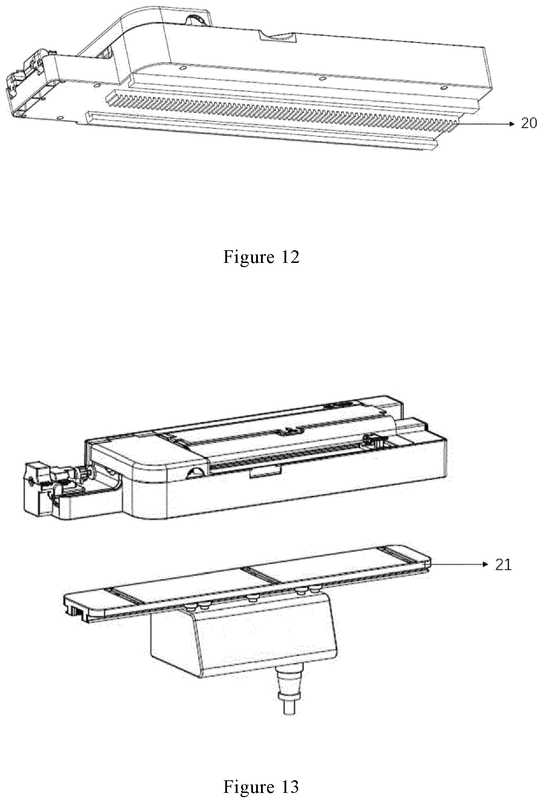

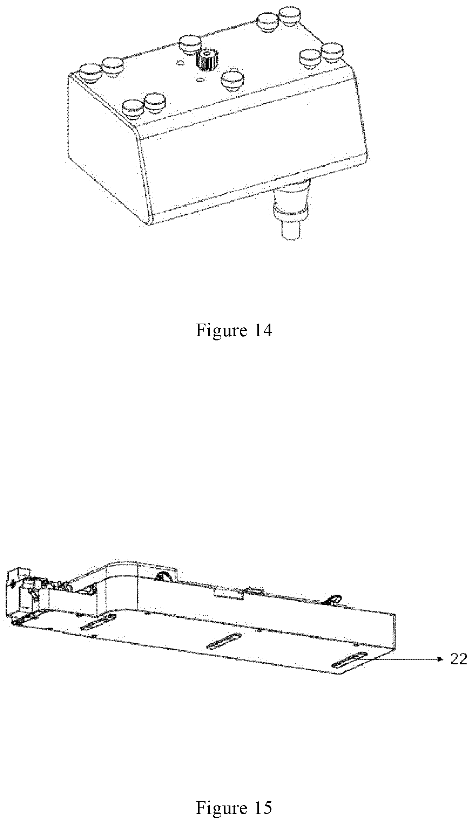

As shown in Figures 13-15, the support frame 1 for the motion system of the vascular intervention navigation surgery system has a horizontal gear at the top, and a support platform 21 above the support frame 1. The bottom of the support platform 21 has a single-sided horizontal rack that matches the horizontal gear. The top of the support platform 21 has a magnetic suction device. The bottom of the vascular intervention navigation surgery system 2 has a magnetic structure 22 that is tightly attached to the support platform 21 and drives the horizontal gear to rotate, Furthermore, it drives the movement of the unilateral transverse rack, thereby causing the vascular intervention navigation surgery system to move.

### Example 6

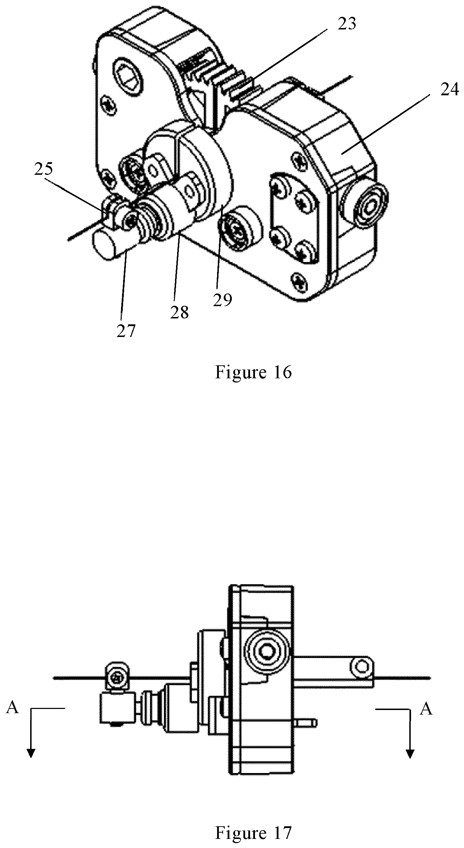

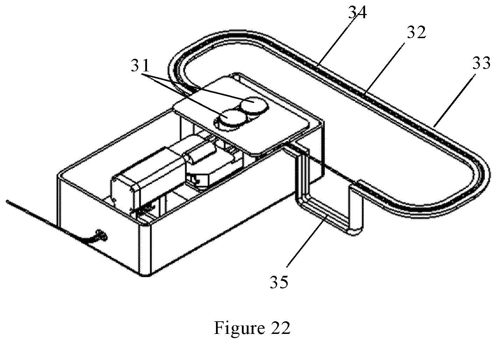

The force reproduction system for the vascular intervention navigation surgery system in this Example is shown in Figures 16-22. The force reproduction system adjunct is fixed to the sun wheel 23, following the sun wheel 23 and then moving forward, backward, and rotating together with the guidewire.

The force reproduction system adjunct of this Example includes a guidewire fixator 25, a pressure sensor, and a sliding sleeve component. The guidewire fixator25 is configured to clamp and fix the guidewire. The pressure sensor includes a pressure sensing plate 26. The sliding sleeve component includes a first sliding kit 27 and a second sliding kit 28. One end of the first sliding kit 27 is fixedly connected to the guidewire fixator 25. The first sliding kit 27 can slide relative to the second sliding kit 28, allowing the other end of the first sliding kit 27 to selectively press against the pressure sensing plate 26. Wherein, the guidewire fixator 25 and the sliding sleeve component are eccentrically set, and the sliding sleeve component is coaxially set with the pressure sensor.

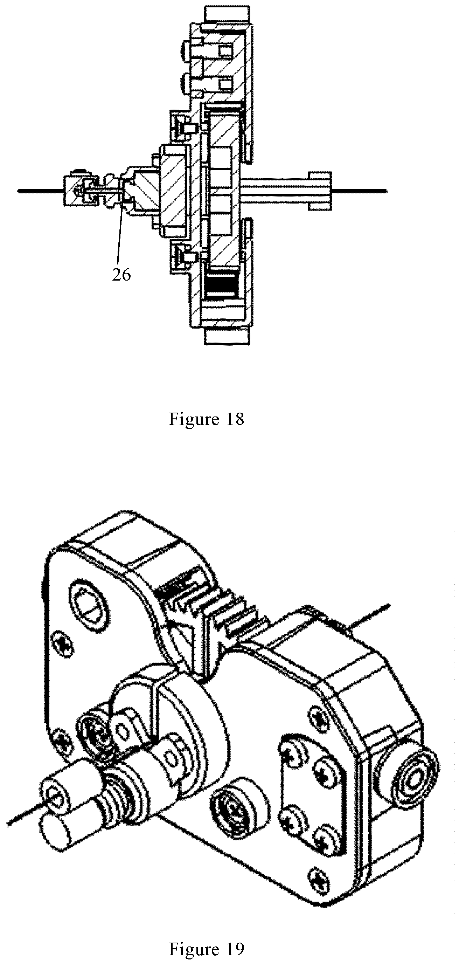

The guidewire fixator 25 can be a clamp structure as shown in Figures 16-18, a guidewire sleeve structure with high friction as shown in Figures 19-20, and so on. There are no special restrictions on the specific structure of the guidewire fixator 25, as long as it can clamp and fix the guidewire.

Preferably, the force reproduction system adjunct may also include an electric induction magnetic coil, wherein the electric induction magnetic coil is attracted to the guidewire fixator 25 and/or the first sliding kit 27.

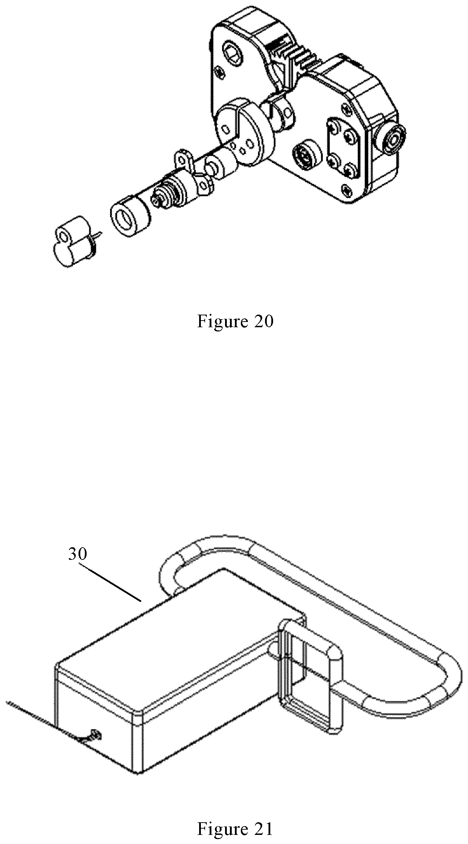

As shown in Figures 21-22, the force reproduction system main end 30 includes a wheel group 31 and a simulated guidewire 32 placed between wheels of the wheel group 31, where the resistance of the wheel group 31 to the simulated guidewire 32 is equal to the resistance experienced by the guidewire during the forward process. That is to say, the resistance of the wheel group 31 to the simulated guidewire 32 is equal to the force value sensed by the pressure sensor.

When the operator feels the resistance of the simulated guidewire 32, they rely on their own experience to determine whether to continue operating the sun wheel 23 to control the guidewire to move forward or rotate. The rear end of wheel group 31 is connected to a motor, which controls the pressure between the wheels of the wheel group 31 through the rotation of the motor, that is, controls the resistance of the simulated guidewire 32 between the wheels of the wheel group 31. In another preferred example, when the motor rotates clockwise or counterclockwise, the pressure between the wheels of the wheel group 31 will increase or decrease, and the resistance of the simulated guidewire 32 between the wheels of the wheel group 31 will increase or decrease.

The force reproduction system main end 30 also includes a simulated pressure sensor and a main end control system. The simulated pressure sensor is configured to sense the operator's thrust on the simulated guidewire 32, in order to apply the thrust to the guidewire. When the operator pushes the simulated guidewire 32 against the resistance of the wheel group 31, the motion of the simulated guidewire 32 drives the rotation of the wheel group 31. Based on the rotation of the wheel group 31 detected by the simulated pressure sensor, it controls the forward or backward movement of the sun wheel 23, thereby driving the forward or backward movement of the guidewire. The conversion from the rotational motion of wheel group 31 to the forward or backward rotation angle of sun wheel group 23 to the linear distance is processed by the main end control system.

The simulated guidewire 32 is supported by guidewire tray 33. The simulated guidewire 32 is connected end-to-end and is cyclically pushed. The guidewire tray 33 can be, but is not limited to, circular, round, or arc-shaped. The guidewire tray 33 has a guidewire groove 34, and the guidewire is embedded in the guidewire groove 34; The guidewire tray 33 is equipped with a guidewire bridge 35. At the guidewire bridge 35, the simulated guidewire 32 separates from the guidewire groove 34, and the operator grasps and pushes the simulated guidewire 32 at the guidewire bridge 35. The length of the guidewire bridge 35 is 1-20 centimeters; Preferred 3-6 centimeters. The diameter of the simulated guidewire 32 is 0.35 millimeters.

The force reproduction system can combine the mechanized vascular intervention navigation surgery system with the experience of clinical doctors. When facing blood vessels with high resistance to pass, it can more effectively operate the guidewire movement and avoid damage to the patient's blood vessels, providing surgical safety.

In addition, the experience values operated by the clinical doctors mentioned above can be numbered and classified, stored in memory for easy recall in the next similar situation, without requiring the clinical doctors to manually operate again.

All documents mentioned in the present invention are cited by reference in this application, just as each document is cited separately as a reference. In addition, it should be understood that after reading the above teachings of the present invention, those skilled in the art can make various changes or modifications to the present invention, and these equivalent forms also fall within the scope defined by the appended claims of this application.

## Claims

1. A motion system for vascular intervention navigation surgery system, wherein the motion system is configured to control the overall motion of the vascular intervention navigation surgery system, the motion system comprises a support base which comprises a support frame and a base, wherein the base provides support for the support frame, and a movable slider is provided on the support frame, and a connecting female head is fixedly installed on the slider;
the vascular intervention navigation surgery system is configured to clamp a medical device which comprises a balloon catheter, a guidewire, and a stent, the vascular intervention navigation surgery system is slidable relative to the support base, and a connecting male head is fixedly installed at the bottom of the vascular intervention navigation surgery system for connection with the connecting female head on the support frame;
the vascular intervention navigation surgery system comprises a Y-connector platform which is configured to accommodate and fix a Y-connector and a guiding catheter connected to the Y-connector; and
the Y-connector is equipped at its distal end with a wheel sleeve which is fixedly connected coaxially with a main channel of the Y-connector, wherein the wheel sleeve is driven by a wheel sleeve driving mechanism to rotate, thereby driving the guiding catheter to rotate.

2. The motion system according to claim 1, wherein an electric motor is installed inside the support frame, and the electric motor drives the slider to move in a straight line on the support frame; after the connecting female head on the slider is paired and connected with the connecting male head at the bottom of the vascular intervention navigation surgery system, the vascular intervention navigation surgery system moves in a straight line with the slider on the support frame.

3. The motion system according to claim 1, wherein an induction device is amounted at top of the support frame, wherein the induction device is configured to sense the position and distance of the connecting male head and/or the connecting female head.

4. The motion system according to claim 1, wherein the base includes a C-shaped slot and a connecting rod;
the connecting rod runs through the C-shaped slot, and the C-shaped slot is configured to move up and down along the connecting rod to adjust height;
the connecting rod is connected to the support frame through a hinge structure, and the support frame is configured to rotate around the connecting rod to adjust angle.

5. The motion system according to claim 1, wherein the wheel sleeve is fixed to a front end of the Y-connector through a polygonal inner hole.

6. The motion system according to claim 1, wherein the vascular intervention navigation surgery system comprises a guidewire limiting structure, which is set between a fixed plate and the Y-connector platform; the guidewire limiting structure comprises a positioning groove and a cover plate, wherein the positioning groove is configured to accommodate the guidewire, and the positioning groove corresponds to a wire groove of the fixed plate, jointly forming a guidewire channel for placing the guidewire; the cover plate is equipped with one or more protruding cover pieces, which can be inserted into the positioning groove so as to cover and reduce an opening area of the positioning groove, thereby limiting the guidewire.

7. A force reproduction system for vascular intervention navigation surgery system, wherein the force reproduction system comprises a force reproduction system adjunct which comprises:
a guidewire fixator configured to clamp and fix a guidewire;
a pressure sensor comprising a pressure sensing plate, and
a sliding sleeve component comprising a first sliding kit and a second sliding kit, wherein one end of the first sliding kit is fixedly connected to the guidewire fixator, and the first sliding kit is configured to slide relative to the second sliding kit so that the other end of the first sliding kit can selectively press against the pressure sensing plate;
wherein the force reproduction system adjunct comprises an electric induction magnetic coil, the electric induction magnetic coil is installed on an outer ring of the pressure sensor, and the electric induction magnetic coil is attracted to the guidewire fixator and/or the first sliding kit.

8. The force reproduction system according to claim 7, wherein the guidewire fixator and the sliding sleeve component are eccentrically set.

9. The force reproduction system according to claim 7, wherein the sliding sleeve component is coaxial with the pressure sensor.

10. The force reproduction system according to claim 7, wherein the guidewire fixator is a sleeve which is a hollow tube that runs through the front and back, and the guidewire passes through the sleeve and is clamped and fixed by an inner wall of the sleeve.

11. The force reproduction system according to claim 7, wherein the force reproduction system further comprises a force reproduction system main end, wherein the force reproduction system main end comprises a wheel group and a simulated guidewire placed between wheels of the wheel group, wherein the resistance of the wheel group against to the simulated guidewire is equivalent to the resistance experienced by the guidewire during a forward process.

12. The force reproduction system according to claim 11, wherein the force reproduction system main end comprises a simulated pressure sensor, which is configured to sense an operator's thrust on the simulated guidewire, so that the thrust is further applied on the guidewire.

13. The force reproduction system according to claim 12, wherein when the operator pushes the simulated guidewire against the resistance of the wheel group, motion of the simulated guidewire drives rotation of the wheel group, and forward or backward movement of a sun wheel group is controlled based on the rotation of the wheel group sensed by the simulated pressure sensor, thereby driving forward or backward movement of the guidewire.

14. The force reproduction system according to claim 11, wherein the simulated guidewire is supported by a guidewire tray.

15. The force reproduction system according to claim 14, wherein the guidewire tray has a guidewire groove, and the simulated guidewire is embedded in the guidewire groove; the guidewire tray is equipped with a guidewire bridge, and the simulated guidewire detaches from the guidewire groove at the guidewire bridge, and the operator grasps and pushes the simulated guidewire at the guidewire bridge.
